# EUROPEAN PATENT APPLICATION

(11) **EP 4 535 369 A1**
(43) Date of publication of application: **09.04.2025**
(21) Application number: 23815945.3
(22) Date of filing: 26.05.2023
(51) Int. Cl.: G16H 50/30

(54) **DISEASE EVALUATION INDICATOR CALCULATION SYSTEM, METHOD, AND PROGRAM**

(30) Priority: 30.05.2022 JP 2022087445
(71) Applicant: Symbiosis Solutions Inc., Tokyo 101-0064 (JP); Japan Agricultural Frontier Development Organization, Tokyo 101-0064 (JP)
(72) Inventor: MASUYAMA Hiroaki, Tokyo 101-0064 (JP); HASUKO Kazumi, Tokyo 101-0064 (JP); TOKUNO Hidetaka, Tokyo 101-0064 (JP); KASUGA Jumpei, Tokyo 101-0064 (JP); ITOGA Tatsuya, Tokyo 101-0064 (JP)
(74) Representative: Marks & Clerk LLP
(86) International application number: PCT/JP2023/019621
(87) International publication number: WO 2023/234188

(57) **Abstract**

A technique for calculating a disease evaluation index using intestinal flora of a user is presented, when the user does not answer questions on the user's disease status in detail. A method for calculating a disease evaluation index, includes inputting a disease that a user wants to evaluate whether it is at risk for; extracting a first intestinal flora of healthy people and a second intestinal flora of people with the disease, using intestinal flora data of a plurality of subjects, and result data of a questionnaire for the subjects; creating an association model including a first observed variable for genera that express a difference in the first intestinal flora and the second intestinal flora, a second observed variable for the disease, and a latent variable relating to the first observed variable; creating a score estimation model including the genera as an explanatory variable, and a score of the latent variable as an objective variable; inputting the user's intestinal flora data to the score estimation model, and estimating the score of the latent variable; and calculating the user's risk for the disease, using a parameter being from the latent variable to the second observed variable and the estimated score.

## Description

### Technical Field

An embodiment of the invention relates to techniques for calculating a disease evaluation index using intestinal flora, and another embodiment relates to techniques for providing an evaluation report that shows a risk for a specific disease to a person based on a stool sample submitted by the person.

### Background

A technique for calculating disease evaluation indexes using intestinal flora of living things, such as humans is being developed. Patent document 1 discloses a technique for providing an evaluation report that shows a risk of a specific disease to a subject who submits a stool sample and takes the time to answer detailed questionnaires about disease statuses.

### Related Art

### Patent Documents

Patent document 1: JP 6533930 B

### Summary

### Technical Problem

However, a disease risk coefficient table needs to be prepared, before calculating a disease evaluation index. The disease risk coefficient table relates to a path between predetermined intestinal flora and a predetermined disease and/or health condition at a predetermined attribute.

One or more embodiments of the instant invention focus on solving such a problem. An object of the embodiments is to provide a technique for calculating a disease evaluation index using intestinal flora of a user, when the user does not answer questions on the user's disease status in detail.

### Solution to Problem

The first embodiment is a disease evaluation index calculation system. The system includes an input unit configured to input one or more diseases that a user wants to evaluate whether it is at risk for into the system; an extraction unit configured to extract a first intestinal flora data of healthy people and a second intestinal flora data of people with the one or more diseases, using a first database that stores intestinal flora data being results of analyzing stool samples of a plurality of subjects, a second database that stores result data of a questionnaire on the one or more diseases for the plurality of subjects, and predetermined extraction conditions; a first creation unit configured to input the first intestinal flora data, the second intestinal flora data, and the result data of the questionnaire, and to create an association model including a first observed variable for one or more genera that express a difference in the first intestinal flora data and the second intestinal flora data, a second observed variable representing a morbidity status of the one or more diseases, one or more latent variables relating to the first observed variable and the second observed variable, and parameters being from the one or more latent variables to the first and the second observed variables and/or being between the latent variables; a second creation unit configured to create a score estimation model including the first observed variable as an explanatory variable, and a score of the latent variable as an objective variable; an estimation unit configured to input the user's intestinal flora data to the explanatory variable of the score estimation model, and to estimate the score of the latent variable; and a calculation unit configured to calculate the user's risk for the one or more diseases, using parameters being from the one or more latent variables to the second observed variables and/or being between the latent variables, and the estimated score.

The second embodiment is the system of the first embodiment, wherein the parameters being from the one or more latent variables to the second observed variables are path coefficients being from the one or more latent variables to the second observed variables.

The third embodiment is the system of the first embodiment, wherein the first observed variable is a genus that is significantly difference in abundance between the first intestinal flora data and the second intestinal flora data.

The fourth embodiment is the system of the first embodiment, wherein the calculation unit uses a logistic regression model, when the second observed variables are binary categorical variables.

The fifth embodiment is the system of the first embodiment, wherein the people with the one or more diseases include people who currently have the one or more diseases and/or people who have previously had the one or more diseases.

The sixth embodiment is the system of the first embodiment, wherein the first creation unit further comprises a selection unit that selects the one or more genera using effect sizes as indexes.

The seventh embodiment is the system of the first embodiment, wherein the second creation unit extracts a measurement equation model that shows influence of the latent variables on the first observed variable, from the association model, and sets each parameter value of the measurement equation model to each parameter value of the score estimation model.

The eighth embodiment is the system of the seventh embodiment, wherein the second creation unit further comprises a learning unit that learns each of the parameter values of the score estimation model using the first intestinal flora data and the second intestinal flora data.

The ninth embodiment is the system of the first embodiment, wherein, when the one or more diseases are plural, the first creation unit creates the association models for each of the diseases, the second creation unit creates the score estimation models for each of the diseases, and the calculation unit calculates the risks for each of the diseases or the risk for the plurality of diseases.

The tenth embodiment is the system of the first embodiment, wherein, when the one or more diseases are plural, the second observed variables are plural, the first creation unit adds another latent variable relating to the plurality of second observed variables to the association model, the calculation unit to estimate score of the other latent variable from the estimated score, and to calculate the user's risk for the plurality of diseases using the parameters being from the other latent variable to the second observed variables and the score of the other latent variable.

The eleventh embodiment is a disease evaluation index calculation method, using a computer. The method includes inputting one or more diseases that a user wants to evaluate whether it is at risk for into the computer; extracting a first intestinal flora data of healthy people and a second intestinal flora data of people with the one or more diseases, using a first database that stores intestinal flora data being results of analyzing stool samples of a plurality of subjects, a second database that stores result data of a questionnaire on the one or more diseases for the plurality of subjects, and predetermined extraction conditions; inputting the first intestinal flora data, the second intestinal flora data, and the result data of the questionnaire, and creating an association model including a first observed variable for one or more genera that express a difference in the first intestinal flora data and the second intestinal flora data, a second observed variable representing a morbidity status of the one or more diseases, one or more latent variables relating to the first observed variable and the second observed variable, and parameters being from the one or more latent variables to the first and the second observed variables and/or being between the latent variables; creating a score estimation model including the first observed variable as an explanatory variable, and a score of the latent variable as an objective variable; inputting the user's intestinal flora data to the explanatory variable of the score estimation model, and estimating the score of the latent variable; and calculating the user's risk for the one or more diseases, using parameters being from the one or more latent variables to the second observed variables and/or being between the latent variables, and the estimated score.

The twelfth embodiment is a program for calculating a disease evaluation index, executed by a computer. The program includes an input step of inputting one or more diseases that a user wants to evaluate whether it is at risk for into the computer; an extraction step of extracting a first intestinal flora data of healthy people and a second intestinal flora data of people with the one or more diseases, using a first database that stores intestinal flora data being results of analyzing stool samples of a plurality of subjects, a second database that stores result data of a questionnaire on the one or more diseases for the plurality of subjects, and predetermined extraction conditions; a first creation step of inputting the first intestinal flora data, the second intestinal flora data, and the result data of the questionnaire, and creating an association model including a first observed variable for one or more genera that express a difference in the first intestinal flora data and the second intestinal flora data, a second observed variable representing a morbidity status of the one or more diseases, one or more latent variables relating to the first observed variable and the second observed variable, and parameters being from the one or more latent variables to the first and the second observed variables and/or being between the latent variables; a second creation step of creating a score estimation model including the first observed variable as an explanatory variable, and a score of the latent variable as an objective variable; an estimation step of inputting the user's intestinal flora data to the explanatory variable of the score estimation model, and estimating the score of the latent variable; and a calculation step of calculating the user's risk for the one or more diseases, using parameters being from the one or more latent variables to the second observed variables and/or being between the latent variables, and the estimated score.

### Advantage

One or more embodiments provide a technique for calculating a disease evaluation index using intestinal flora of a user, when the user does not answer questions on the user's disease status in detail.

### Brief Description of Drawings

Fig. 1 is a block diagram of a disease evaluation index calculation system in accordance with one or more embodiments.
Fig. 2 is a flowchart of calculating a disease evaluation index in accordance with the embodiment.
Fig. 3 is a table of conditions for the normal control group (NC).
Fig. 4 is a table of conditions for the patients with atopic dermatitis without other diseases (AS).
Fig. 5 is a table of conditions for the patients with atopic dermatitis and other diseases (AM).
Fig. 6 is a table of conditions for the patients without atopic dermatitis but with other diseases (OD).
Fig. 7 is an explanatory diagram of selecting Genus variables.
Fig. 8 is an explanatory diagram of the transition in numbers of genera that are candidates for the selection.
Fig. 9 shows a result of estimating parameters of the association model in accordance with the first embodiment.
Fig. 10 shows a result of ROC analysis of risk value for atopic dermatitis.
Fig. 11 shows an outline of a plural of association models in accordance with the second embodiment.
Fig. 12 shows an outline of another association model in accordance with the third embodiment.

### Detailed Description of Embodiments

One or more embodiments of the invention are described with reference to the drawings. The same reference numerals are given to common parts in each figure, and duplicate description is omitted.

### (Disease evaluation index calculation system)

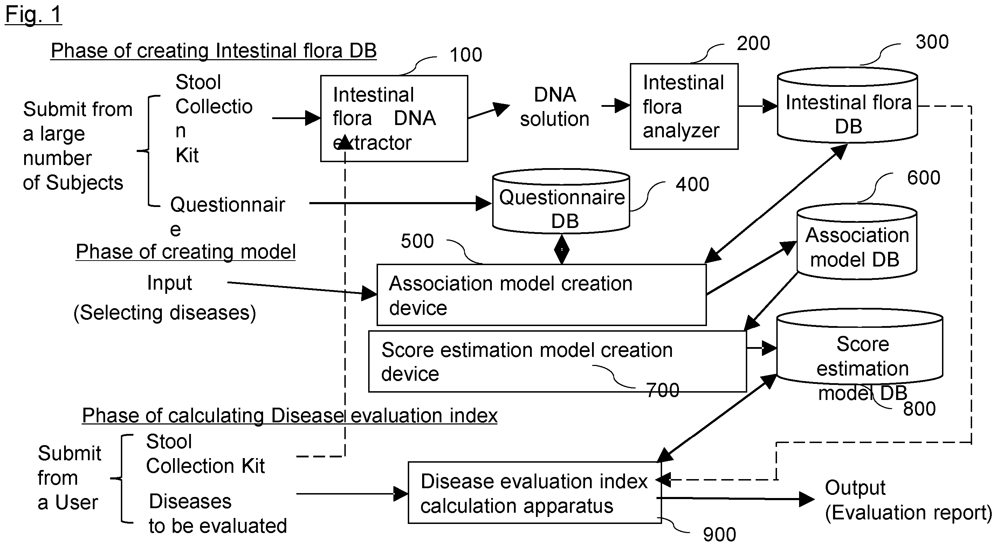

Fig. 1 is a block diagram of a disease evaluation index calculation system in accordance with one or more embodiments. The disease evaluation index calculation system includes a phase of creating an intestinal flora database (DB) from stool collection kits submitted by a large number of subjects, a phase of creating a model relating to one or more diseases (a specific disease), and a phase of calculating an evaluation index of a risk for the specific disease.

### (Phase of creating Intestinal flora DB)

In the phase of creating Intestinal flora DB, ten thousand or more of subjects excrete their stools in toilets and collect their stool samples with stool collection kits. Next, an extractor received the stool collection kits. The extractor inputs the stool collection kits into an intestinal flora DNA extractor 100, and outputs a DNA solution relating to intestinal flora. Intestinal flora is also called as microbiota, human gut microbiota, or human intestinal microbiome.

An analyst who received the DNA solution inputs the DNA solution into an intestinal flora analyzer 200 and analyzes the intestinal flora. The analyst stores the analysis result data of the intestinal flora in an intestinal flora DB 300. First, the embodiment creates ASV (Amplicon Sequence Variants) sequences that are unique to each subject's intestinal flora. Second, the embodiment identifies a genus name for each subject's intestinal flora data using an external DNA sequence database (for example, SILVA rRNA database) that is publicly available.

The intestinal flora DB 300 associates the ID (Identification number) information of each subject with the intestinal flora of the subject. Although the extractor and the analyst have been described separately, the same business operator may perform the extraction work and the analysis work.

Next, the large number of subjects who submitted the stool collection kits fill out questionnaires and submit their questionnaire results to a questionnaire collector, using the ID information. The questionnaire collector stores the questionnaire results in a questionnaire DB 400.

A questionnaire survey gathers the subjects' detailed information, including the subjects' attributes (gender and age), Body Mass Index (BMI), defecation frequency, lifestyle (drinking, smoking, exercise frequency), disease morbidity (including disease status), sleep status, the Center for Epidemiologic Studies Depression scale (CES-D), presence or absence of Helicobacter pylori treatment, hospitalization or surgery experience, and, status for prescription or over-the-counter medications. For females only, the questionnaire survey gathers their menstrual status and whether they were pregnant or breastfeeding. Disease morbidity was divided into 15 categories (atopic dermatitis, bone and/or joint disease, bronchial asthma, diabetes, dyslipidemia, gastro-intestinal disease, heart disease, hypertension, kidney disease, liver disease, lower back and/or joint pain, and the others) and identified for each patient, including whether or not they were currently being treated. A patient suffering from a disease includes a patient who currently has the disease and/or a patient who has previously had the disease. Prescription or over-the-counter medication use was divided into 16 categories (gastric/duodenal ulcer/reflux esophagitis, hypertension, hyperlipidemia, diabetes, hypnotics, painkillers/antipyretics, allergy, angina treatment, laxative/constipation, osteoporosis, rheumatism, corticosteroids, antibiotics, cold drugs, antithrombotic drugs, and the others) and identified for each patient, including whether or not they were currently being treated at the time of stool collection.

### (Phase of creating model)

In the model creation phase, first of all, the model creator selects one or more diseases to be evaluated. An association model creation device (or an association modeling device) 500 includes input unit that inputs the selected diseases, an extraction unit that extracts ID information of subjects related to the diseases from the questionnaire DB 400 for each attribute of male and female, and the creation unit that creates an association model using a creation method described below. The model creation device 500 stores the created association model in an association model DB 600.

Next, the model creator extracts the association model from the association model DB 600 and creates a score estimation model using a creation method described later. The created score estimation model is stored in a score estimation model DB 800.

### (Phase of calculating Disease evaluation index)

In the disease evaluation index calculation phase, an evaluator requests a person, who submits the person's stool sample collected by the stool collection kits and wishes, hopes or desires to evaluate the risk for the specific disease (hereinafter referred to as "a user"), to submit the user's stool collection kit explained in the phase of creating Intestinal flora DB. The user's stool collection kit is processed by the intestinal flora DNA extractor 100 and the intestinal flora analyzer 200, in the same way as the stool collection kits of the large number of subjects. The intestinal flora data of the user is stored in the intestinal flora DB 300. Furthermore, the evaluator input the specific disease that the user wishes to evaluate and the user's ID information (including the user's attributes, such as the user's gender) to a disease evaluation index calculation apparatus 900.

The disease evaluation index calculation apparatus 900 includes an input unit that receives input information, an extraction unit that extracts information related to the input information, a calculation unit that calculates the evaluation index, and an output unit that outputs an evaluation report (or an assessment report). The input unit accepts input of the specific disease and the user's ID information as the input information. The extraction unit extracts information related to the user's ID information from the external intestinal flora DB 300 and extracts a score estimation model related to the specific disease from the score estimation model DB 800. The disease evaluation index calculation apparatus 900 inputs the intestinal flora data of the user to the score estimation model related to the specific disease and calculates the evaluation index of the risk for the specific disease. An evaluation report is output based on the calculated evaluation index. Although business operators who perform each phase have been described separately, the same business operator may perform all the phases.

### (Flowchart of calculating a disease evaluation index)

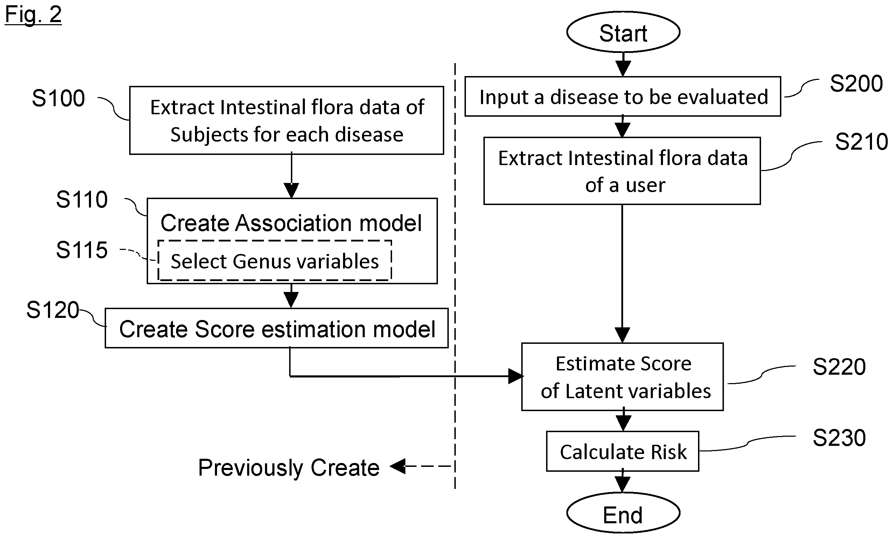

Fig. 2 is a flowchart of calculating a disease evaluation index in accordance with the embodiment. These processes are divided into two process groups. The first process group (S100 through S120) creates score estimation models for each disease in advance. The second process group (S200 through S240) calculates the evaluation index of the risk for the specific disease that the user wishes to evaluate.

The first process group includes a step of extracting intestinal flora data of a plurality of subjects for each disease (S100), a step of creating an association model for each disease (S110), and a step of creating a score estimation model for each disease (S120). The second process group includes a step of inputting a disease that a user wishes to evaluate (S200), a step of extracting intestinal flora data of the user (S210), a step of inputting the intestinal flora data of the user to the score estimation model for a specific disease and estimating score of latent variables (S220), and a step of calculating an evaluation index of the user's risk for the specific disease (S230).

### (Description of S100)

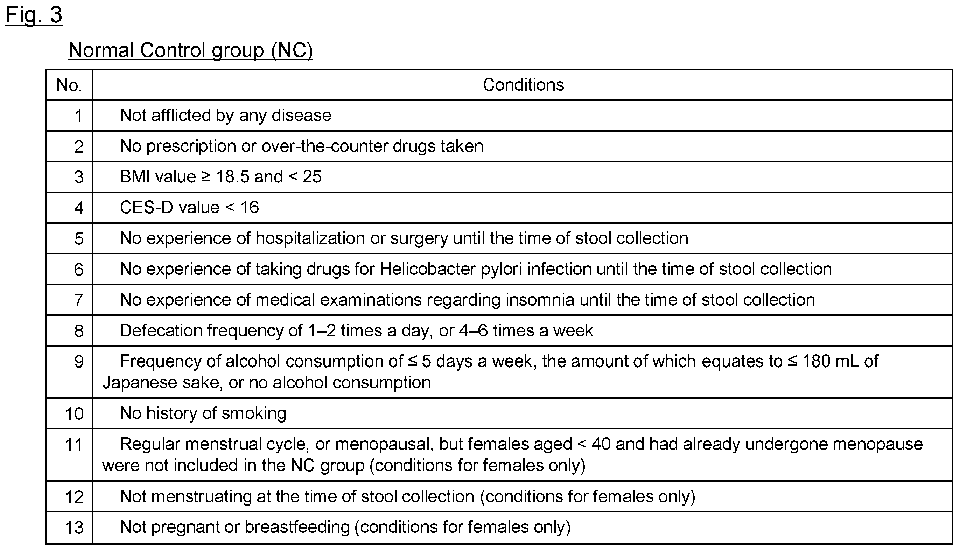

**In** the embodiment, "Atopic dermatitis" is described as an example of the diseases. Hereinafter "Atopic dermatitis" is also referred to as "Atopy". S100 extracts gut microbiota data of a group of healthy participants and a group of patients afflicted with atopic dermatitis from gut microbiota DB. **In** the embodiment, healthy participants and patients afflicted with diseases are included in the plurality of subjects. Healthy participants are called as healthy people. Patients afflicted with diseases are called as people with diseases. Fig. 3 through Fig. 6 show conditions of extracting each group. These conditions include conditions for females only.

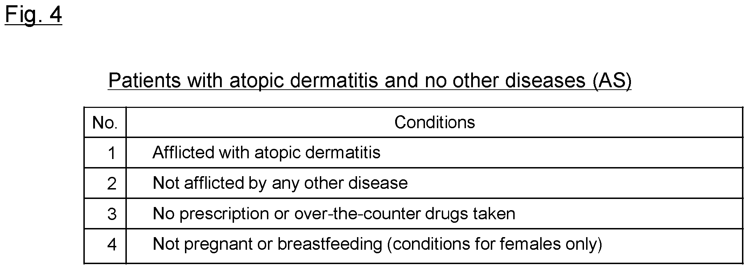

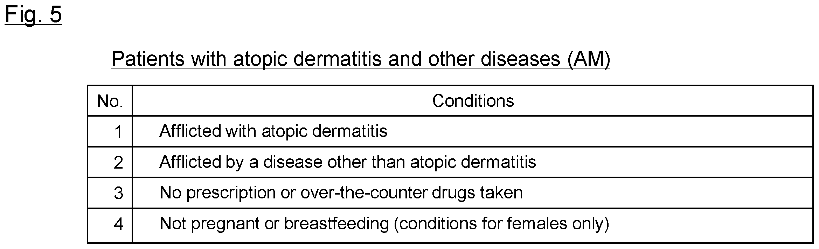

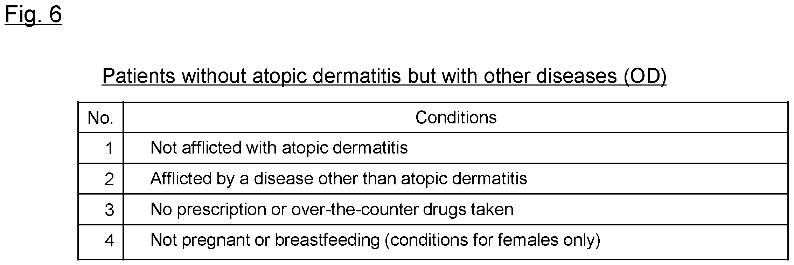

Fig. 3 is a table of conditions for the normal control group (NC). The normal control group (Normal Control, hereinafter referred to as NC) is a group of healthy participants. The NC consists of participants who satisfied all conditions shown in Fig. 3. Fig. 4 is a table of conditions for the patients with atopic dermatitis without other diseases (AS). The patients with atopic dermatitis without other diseases (Atopic dermatitis Single, hereinafter referred to as AS) is a group of participants who satisfied all conditions shown in Fig. 4. Fig. 5 is a table of conditions for the patients with atopic dermatitis and other diseases (AM). The patients with atopic dermatitis and other diseases (Atopic dermatitis Multi, herein after referred to as AM) is a group of participants who satisfied all conditions shown in Fig. 5. Fig. 6 is a table of conditions for the patients without atopic dermatitis but with other diseases (OD). The patients without atopic dermatitis but with other diseases (Other Disease, herein after referred to as OD) is a group of participants who satisfied all conditions shown in Fig. 6. In the embodiment, each group are furthermore divided into male and female. The following describes a case of female groups.

### (Description of S110)

S110 is a step of creating an association model for atopic dermatitis. The embodiment uses the female groups and creates the association model for the female atopic dermatitis. First, the gut microbiota data extracted in S100 is converted using a Centered Log-Ration (CLR) transformation. The count values of each genus in the gut microbiota data are converted into the CLR values. Processing this transformation makes the following statistical process easier.

Next, the embodiment performs a step of selecting genus variables (S115). S115 is performed to search for genera that express differences in the gut microbiota of NC and AS. The embodiment selects genera using the effect size between NC and AS as an index. The effect size was calculated 500 times. Parameters for calculating the effect size are set to select genera that are ranked within the top of 20 in all trials of the calculation, when arranging in descending order based on the absolute value of the effect size.

There are three reasons that the groups to be selected for genus variables are set to two groups of NC and AS. The first reason is that if there are patients with diseases other than atopic dermatitis in the atopic dermatitis group, the genera affected by those diseases will also be selected. The genera may affect the accuracy of creating models, therefore AS is set instead of AM. The second reason is that if the conditions of NC are simply to be not afflicted with atopic dermatitis, the genera affected by other diseases will also be selected. The genera that characteristically express the difference between the gut microbiota in a healthy state and the gut microbiota in atopic dermatitis state may not be selected, therefore the NC is set as a group of healthy participants. The third reason is that the use of drugs may affect the gut microbiota. In patients who take some drugs, the state of the gut microbiota, which is characteristic of patients afflicted with atopic dermatitis, may be disturbed. Therefore, OD will not be used.

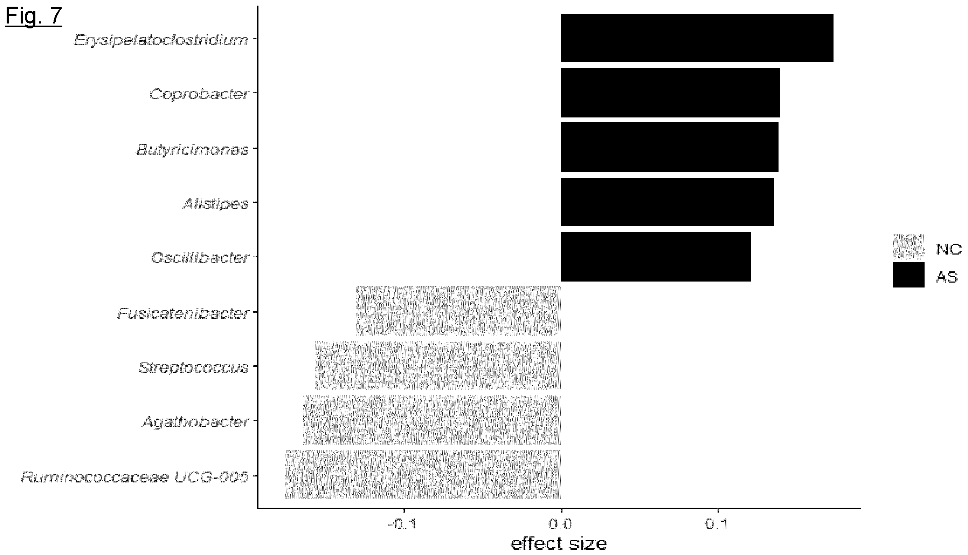

Fig. 7 is an explanatory diagram of selecting Genus variables. The effect size value is the average value of the results of the 500 calculations. Five genera with high positive effect sizes are selected for AS. Four genera with high negative effect sizes are selected for NC. Thus, the genera with positive effect sizes tend to transit from a healthy state of the gut microbiota to a state afflicted with atopic dermatitis, while the genera with negative effect sizes tend not to transit.

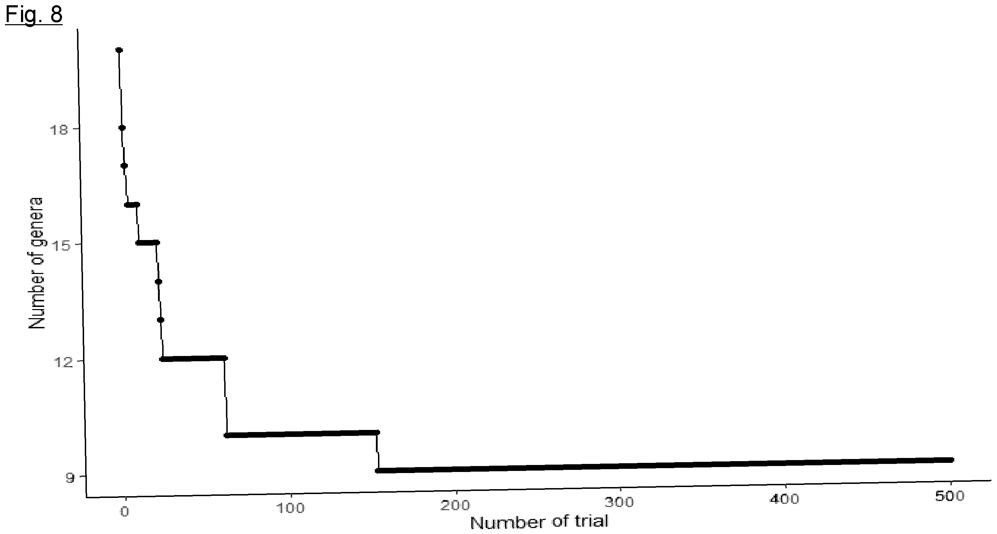

Fig. 8 is an explanatory diagram of the transition in numbers of genera that are candidates for the selection. The horizontal axis shows the number of trials conducted to calculate effect sizes. The vertical axis shows the number of genera that continue to be in the top of 20 until the number of trials of the horizontal axis, when arranging in descending order based on the absolute value of the effect size.

Returning to the process of S110, the step of creating an association model using the genera selected in S115 is described below. The embodiment creates the association model using the genera that are converted into the CLR values and the Atopy morbidity data. The genera and the Atopy morbidity data are related to NC and AS.

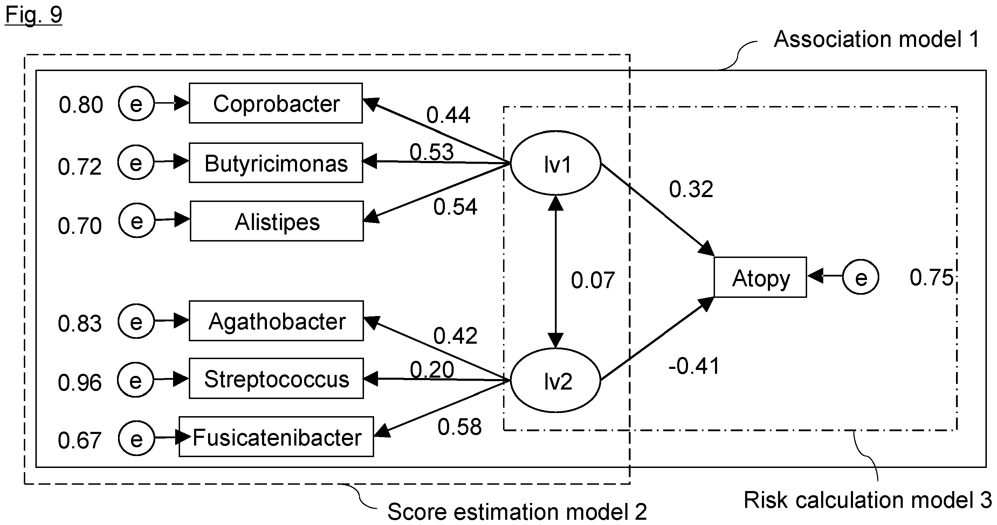

Fig. 9 shows a result of estimating parameters of the association model in accordance with the first embodiment. The embodiment uses Structural Equation Model (SEM) as an association model. SEM represents relationships between observed variables and latent variables, and relationships between the latent variables. Therefore, the association model is a statistical analysis model constructed by observed variables that have a certain degree of correlation with a specific disease and latent variables that are related to the observed variables.

The association model 1 is an area surrounded by the solid line. Rectangles show the observed variables. "e" is a residual variance. Ellipses show the latent variables. Numerical values of arrows that are from the latent variables to the observed variables or the other latent variables show standardized parameters of the association model. For example, the observed variable "Atopy" is an observed variable for the disease "atopic dermatitis" and is defined as binary categorical variables representing disease morbidity of atopic dermatitis. When a patient is afflicted with atopic dermatitis, the observed variable "Atopy" is "1 (one)." When the other patient is not afflicted with atopic dermatitis, the observed variable "Atopy" is "0 (zero)."

The embodiment assumes that there are two latent variables that explained the binary categorical variables, in case of creating the association model. The latent variable 1 (lv 1) has a positive effect on Atopy. The latent variable 2 (lv 2) has a negative effect on Atopy.

First, all of the genera shown in Fig. 7 are assigned as observed variables related to latent variables Lv1 or Lv2. This is the first hypothesized association model. The embodiment uses the first hypothesized association model as a starting point to modify the model. The step of modifying the model conducts to delete observed variables until no negative components appeared in the variance-covariance matrix calculated in the process of the structural equation modeling.

Subsequently, observed variables are deleted so that p-values (significance probability) of each parameter is less than 0.05. The final association model is selected from the constructed models, on the condition that the values of Goodness-of-Fit Index (GFI) and Adjusted GFI (AGFI) are close to "1", the value of Root Mean Square Error of Approximation (RMSEA) is close to "0", and, the absolute value for the path coefficient from the latent variable to the disease morbidity variable representing disease morbidity is maximum. As a result of modifying the model, Erysipelatoclostridium, Oscillibacter, and Ruminococcaeae UCG-005, among the genera shown in Fig. 7, were excluded from the observed variables of the model.

The embodiment merges the data for the NC and AS groups as the genera data and the Atopy morbidity data. The embodiment input the merged data to the created association model and conducts to calculate each parameter of the association model. The following (referring to Fig. 9) investigates how the genera that are set as the observed variables explains the variables representing disease morbidity of atopic dermatitis, using the assumed latent variables (Lv1, Lv2), when focusing on healthy and atopic dermatitis-afflicted individuals.

The standardized path coefficient from latent variable Lv1, which is hypothesized to have a positive effect on Atopy, to the Atopy morbidity variable is 0.32 (p<0.01). The standardized path coefficient from latent variable Lv2, which is hypothesized to have a negative effect on Atopy, to the Atopy morbidity variable is -0.41 (p<0.01). The standardized factor loading values from the latent variable Lv1 to each of the observed variables of Lv1 are 0.54 for Alistipes, 0.53 for Butyricimonas, and 0.44 for Coprobacter. The standardized factor loading values from the latent variable Lv2 to each of the observed variables of Lv2 are 0.58 for Fusicatenibacter, 0.42 for Agathobacter, and 0.20 for Streptococcus. The standardized factor loading values form the latent variables to each of the observed variables are all significant at p<0.05. The numerical value of the double-headed arrow between the latent variables that is 0.07 represents the correlation coefficient.

The residual variance of the Atopy morbidity variable is 0.75, and the association model 1 shows the result that the assumed latent variables (Lv1, Lv2) explain approximately 25% of the variance in the Atopy morbidity variable.

The groups to be objects for selecting genus variables (S115) are set to the two groups of NC and AS, in case of creating the association model 1 shown in Fig. 9. The embodiment compares the latent variable scores in the created association model with other groups.

The latent variable scores of each participant are calculated from each parameter of the association model 1 shown Fig. 9 and compared between groups. The score of Lv 1 is not significantly difference between the NC and OD (p=0.27), while the score for the AS is significantly higher than that for the NC (p<0.01). The score for the AM tends to be higher than that for the NC, however there is no significant difference observed (p=0.12). There is no significant difference observed in the score of Lv 2 for between the NC and OD (p=0.55), and the scores for the AS and AM are significantly lower than that for the NC (p<0.01).

In the embodiment, S115 is an example of a method for selecting observed variables for an association model and selects genus variables using effect sizes as indexes. S115 may not be required.

There are various ways to select observed variables for an association model. For example, some statistical test (such as Wilcoxon rank sum test) may be performed between the normal control group and patients with a disease, and a genus that is significantly difference in abundance between two groups may be used as an observed variable in an association model. Alternatively, a genus that has already been reported to be a potential biomarker for a disease to be evaluated may be used as an observed variable in an association model.

### (Description of S120)

S120 is a step of creating a model for estimating scores of latent variables when a disease morbidity is unknown. The embodiment uses the merged date for the NC and AS groups (that is, the genera data and the Atopy morbidity data), in case of creating an association model. However, a user wishes to evaluate a risk for a specific disease, the user's disease morbidity is unknown. Therefore, the embodiment needs a model for estimating scores of latent variables when a disease morbidity of a specific disease is unknown.

When a female user's disease morbidity of atopic dermatitis is unknown, the embodiment extracts a part of a measurement equation model (surrounded by the dotted line in Fig. 9) from the association model 1 and creates a score estimation model 2, in order to estimate scores of Lv 1 and Lv 2. Therefore, the embodiment sets each parameter value of the measurement equation model to each parameter value of the score estimation model 2. The measurement equation model shows the influence of the latent variables on the observed variables of the genera.

The embodiment estimates the user's scores of Lv 1 and Lv2 from the score estimation model 2. The estimated scores of the latent variables shows a similar trend of significantly difference in comparing groups of the estimated scores latent variables to that of the association model 1. In the following, the estimated values of the latent variables Lv1 and Lv2 are represented as Lvlest and Lv2est, respectively.

### (Description of S200)

S200 is a step of inputting a disease that a user wishes to evaluate. The embodiment creates score estimation models for atopic dermatitis separately for gender. The user also inputs the user's gender. In the embodiment, the user is female.

### (Description of S210)

S210 is a step of extracting gut microbiota data of the user. The user submits the stool collection Kit of the user, and the gut microbiota data of the user is stored in the gut microbiota DB 300. S210 extracts the gut microbiota data of the user from the gut microbiota DB 300 using the user's ID information.

### (Description of S220)

S220 is a step of estimating scores of latent variables using the score estimation model 2 for atopic dermatitis. The embodiment inputs the user's gut microbiota data to the observed variables of the score estimation model 2 for atopic dermatitis and estimates the user's score of the latent variables of the model 2.

### (Description of S230)

S230 is a step of calculating an evaluation index of the risk for atopic dermatitis (or calculating the evaluation index numerically representing the risk for atopic dermatitis). The embodiment calculates the user's risk for atopic dermatitis, using the path coefficients from the latent variables of Lv 1 and Lv 2 to the observed variable "Atopy" shown in Fig. 9 and the estimated values of the latent variables Lv1 and Lv2 (Lvlest and Lv2est). The dash-dotted line shown in Fig. 9 represents a part of the risk calculation model 3.

### (An example of calculating risk)

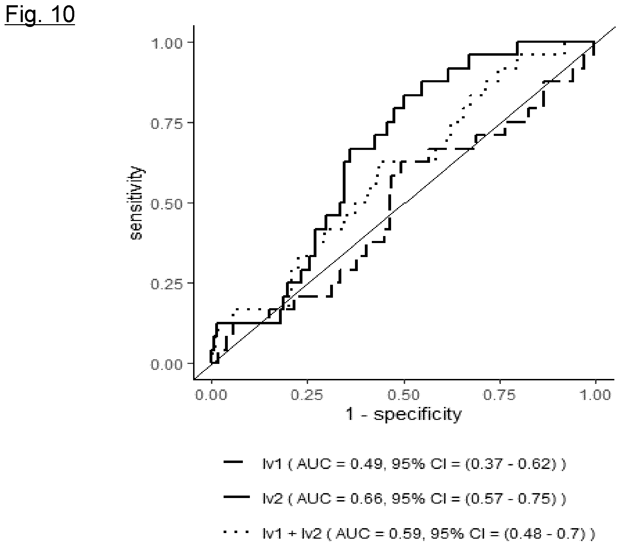

Fig. 10 shows a result of ROC analysis of risk value for atopic dermatitis. The following describes the risk calculation (or calculating a numerical value for the risk) of the embodiment and the accuracy of the calculated risk.

The merged groups of the NC, OD, AS, and AM in female subjects are randomly divided in a stratified manner into 80% as a training group and 20% as a verification group. The embodiment learns an association model using scores of latent variables (Lv1 or Lv2) and atopic dermatitis morbidity data in the training group and constructs an atopic dermatitis morbidity probability estimation model that estimates atopic dermatitis morbidity probability from the scores of the latent variables. The embodiment applies the estimated values of the latent variable scores (Lvlest or Lv2est) to the constructed atopic dermatitis morbidity probability estimation model and investigates the accuracy of the atopic dermatitis morbidity probability estimation model using ROC (Receiver Operating Characteristic) analysis. The atopic dermatitis morbidity probability is a risk for getting atopic dermatitis, and the result of the ROC analysis is used as a disease evaluation index.

The method for constructing the atopic dermatitis morbidity probability estimation model uses a logistic regression model that has latent variables Lv1 and/or Lv2 as explanatory variables, and binary categorical variables representing a user's disease morbidity of atopic dermatitis as objective variables. The logistic regression model corresponds to the risk calculation model 3 in Fig. 9.

Fig. 10 shows ROC curves for the logistic regression model. The vertical axis shows "Sensitivity". As the probability of identifying a patient who actually suffers from a disease as one who has the disease becomes higher, the value on the vertical axis approaches "1". The horizontal axis shows "1 - Specificity". As the probability of identifying a patient who does not suffer from atopic dermatitis as one who does not have atopic dermatitis becomes higher, the value of "Specificity" approaches "1". The horizontal axis represents "1 - (minus) Specificity". As the value on the horizontal axis approaches "0", the probability of a patient who does not suffer from atopic dermatitis as one who does not have atopic dermatitis identifying becomes higher. The dashed line represents the results of the model in which only Lv1 is an explanatory variable, and the thick solid line represents the results of the model in which only Lv2 is an explanatory variable. The dotted line represents the results of the model with Lv1 and Lv2 as explanatory variables. "AUC" represents a value of "Area Under the Curve" and "95% CI" represents the 95% Confidence Interval of the AUC.

In Fig. 10, the diagonal line (thin solid line) represents a probability of "0.5". In the case of the model using only Lv2 (thick solid line), the area enclosed by the thick solid line and the thin solid line is larger than those of the other models. That can be visually (or intuitively) determined that the accuracy of the risk estimation method is high. AUC is an index of the risk estimation method. When AUC value is close to 1, that can be objectively determined to be a good risk estimation method. The AUC of the model using only Lv2 shows the highest value of "0.66". The embodiment is judged to be highly accurate as an indirect risk estimation method, excluding direct risk estimation methods such as blood tests.

### (Effects)

According to the embodiment, the association model for each disease (the association model 1 in Fig. 9) is created using the intestinal flora data and disease morbidity data of the large number of subjects, and the score estimation model (the score estimation model 2 in Fig. 9) is created in advance from a measurement equation portion of the association model. The embodiment estimates scores of latent variables for the user who wishes to evaluate the risk for the specific disease, using the score estimation model for the specific disease and the intestinal flora data of the user. The embodiment inputs the estimates scores of latent variables to the risk calculation model (the risk calculation model 3 in Fig. 9) and calculates the user's risk for the specific disease.

When the user collects and submits the user's stool sample using the stool collection kit, without answering detailed questionnaires about the user's disease morbidity, the user easily receives the evaluation report that shows the risk of the specific disease to the user. The business operator who provides the evaluation report may design, study, and propose probiotics to the user.

### (Variation 1 of the embodiment; When values of each parameter of the score estimation model is learned)

The above embodiment extracts the measurement equation model, that shows the influence of the latent variables on the observed variables of the genera, from the association model, and sets each parameter value of the measurement equation model to each parameter value of the score estimation model. Therefore, the embodiment sets each parameter value of the measurement equation model to each parameter value of the score estimation model, without learning. In contrast, in the variation 1 (or the modified example 1), the values of each parameter of the score estimation model is learned using the intestinal flora data of the NC and AS.

The learning process includes the following 6 steps. (1) The merged groups of the NC and AS in female subjects are randomly divided in a stratified manner into 80% as a training group and 20% as a verification group. (2) For each subject in the training group, the scores of the latent variables (Lv1 or Lv2) of the association model are calculated. (3) For the training group, the calculated scores of the latent variables, the intestinal flora data of the selected genera, and the atopic dermatitis morbidity data are totaled up, and the totaled data are balanced. (4) A linear multiple regression model is created in which the latent variables (Lv1 and Lv2) in the balanced training group are set as objective variables and the intestinal flora data of the genera are set as explanatory variables. (5) Scores of the latent variables (Lv1 and Lv2) in the verification group are estimated using the linear multiple regression model. (6) Scores the latent variables (Lv1 and Lv2) in the verification group calculated from each parameter of the association model are compared with the estimated scores the latent variables (Lv1 and Lv2).

### (Variation 2 of the embodiment; When an association model is created for each disease)

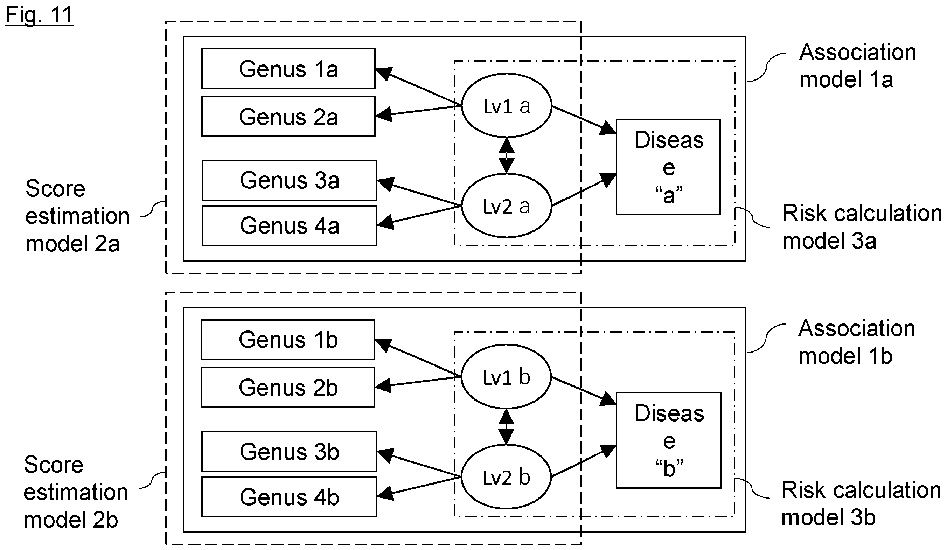

Fig. 11 shows an outline of a plural of association models in accordance with the second embodiment (the variation 2 of the embodiment). In this figure, two association models are used, and numerical values of each arrow are omitted for the sake of simply drawing.

In the above embodiment, the case has been described in which the user wishes to evaluate the user's risk for the specific disease. However, there is another case when another user wishes to evaluate the other user's risks for a plurality of diseases. For example, the other case is when a disease "a" is atopic dermatitis and the other disease "b" is asthma. In the other case, association models (1a, 1b) are created for each disease, score estimation models (2a, 2b) are created for each disease, and risk calculation models (3a, 3b) are also created. In the variation 2, the disease evaluation index calculation system calculates risks for each disease or a risk for a plurality of diseases.

For the risk for the plurality of diseases, for example, the maximum value among the risk values for each disease may be recorded in the evaluation report as the highest risk. Furthermore, the categorical variables representing the morbidity status of each disease are set to binary (or two values) and classified into two groups, but this is not limited and the variables may be classified into three or more groups. For example, when a user wishes to evaluate a risk of atopic dermatitis and asthma, each of the disease morbidity status variables of the association model (a, b) may be combined and the user may be classified into groups, the user without atopic dermatitis and asthma as the group "0", the user with only atopic dermatitis as the group "1", the user with only asthma as the group "2", and the user with both atopic dermatitis and asthma as the group "3".

### (Variation 3 of the embodiment; When latent variables for diseases are used)

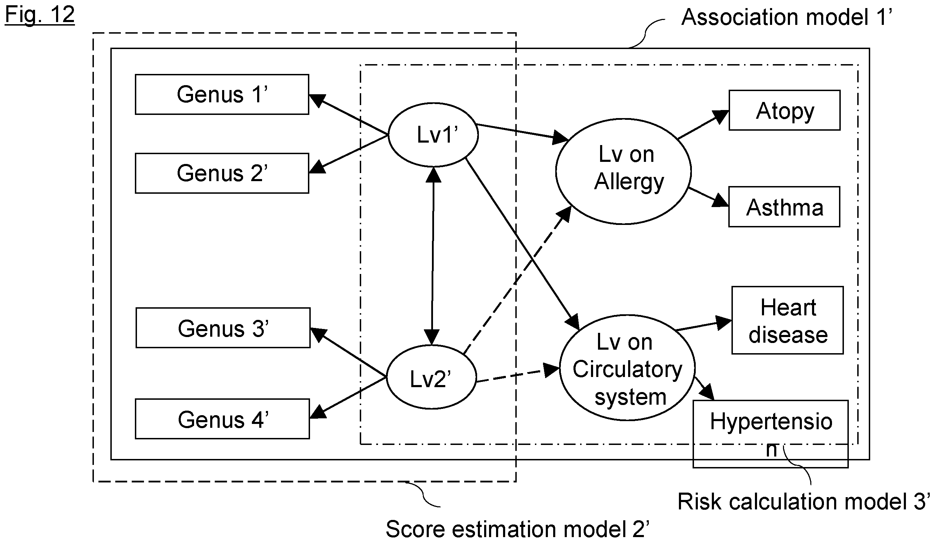

Fig. 12 shows an outline of another association model in accordance with the third embodiment (the variation 3 of the embodiment). In this figure, latent variables being set a plurality of disease morbidity status variables as observed variables is used for an association model, and numerical values of each arrow are omitted for the sake of simply drawing.

"Atopy" and "Asthma" are classified as allergy-related diseases, and "Heart disease" and "Hypertension" are classified as circulatory system-related diseases. A latent variable related to diseases ("Atopy" and "Asthma") are referred to as "Lv on Allergy", and another latent variable related to diseases ("Heart disease" and "Hypertension") are referred to as "Lv on Circulatory system".

An association model 1' in Fig. 12 is a model in which "Lv on Allergy" and "Lv on Circulatory system" are newly added to the association model 1 in Fig. 9. When the association model 1' in Fig. 12 is constructed, a score estimation model 2' and a risk calculation model 3' are created in the same manner as described in Fig. 9.

When a user wishes to evaluate the user's risk for one or more of "Atopy", "Asthma", "Heart disease", and "Hypertension", the embodiment inputs the user's intestinal flora data to the score estimation model 2' and estimates scores of latent variables related to genera (Lv1' and Lv2'). Next, the embodiment estimates scores of the latent variables ("Lv on Allergy" and "Lv on Circulatory system") related to the diseases of the risk calculation model 3'. Finally, the embodiment calculates the risk for the disease that the user wishes to evaluate, using the path coefficients from "Lv on Allergy" to the observed variables ("Atopy" and "Asthma"), the path coefficients from "Lv on Circulatory system" to the observed variables ("Heart disease" and "Hypertension"), and the estimated scores of the latent variables ("Lv on Allergy" and "Lv on Circulatory system").

The above-mentioned embodiments (including modified examples) of the invention have been described. Furthermore, two or more of the embodiments may be combined. Alternatively, one of the embodiments may be partially implemented. Furthermore, two or more of the embodiments may be partially combined.

Furthermore, embodiments of the invention are not limited to the description of the above embodiments. Various modifications are also included in the embodiments of the invention as long as a person skilled in the art can easily conceive without departing from the description of the embodiments.

For example, a number of latent variables is assumed to be 2 (two). the number may be 1 (one), or 3 (three) or more. The embodiment extracts ID information of subjects related to a disease from the questionnaire DB 400 for each attribute of male and female and creates the association model. However, the extraction for each attribute of male and female may not be necessary depending on the type of diseases.

### Reference Signs List

- 100: Intestinal flora DNA extractor
- 200: Intestinal flora analyzer
- 300: Intestinal flora DB
- 400: Questionnaire DB
- 500: Association model creation device
- 600: Association model DB
- 700: Score estimation model creation device
- 800: Score estimation model DB
- 900: Disease evaluation index calculation apparatus

## Claims

1. A disease evaluation index calculation system, comprising:
an input unit configured to input one or more diseases that a user wants to evaluate whether it is at risk for into the system;
an extraction unit configured to extract a first intestinal flora data of healthy people and a second intestinal flora data of people with the one or more diseases, using a first database that stores intestinal flora data being results of analyzing stool samples of a plurality of subjects, a second database that stores result data of a questionnaire on the one or more diseases for the plurality of subjects, and predetermined extraction conditions;
a first creation unit configured to input the first intestinal flora data, the second intestinal flora data, and the result data of the questionnaire, and to create an association model including a first observed variable for one or more genera that express a difference in the first intestinal flora data and the second intestinal flora data, a second observed variable representing a morbidity status of the one or more diseases, one or more latent variables relating to the first observed variable and the second observed variable, and parameters being from the one or more latent variables to the first and the second observed variables and/or being between the latent variables;
a second creation unit configured to create a score estimation model including the first observed variable as an explanatory variable, and a score of the latent variable as an objective variable;
an estimation unit configured to input the user's intestinal flora data to the explanatory variable of the score estimation model, and to estimate the score of the latent variable; and
a calculation unit configured to calculate the user's risk for the one or more diseases, using parameters being from the one or more latent variables to the second observed variables and/or being between the latent variables, and the estimated score.

2. The system of claim 1, wherein the parameters being from the one or more latent variables to the second observed variables are path coefficients being from the one or more latent variables to the second observed variables.

3. The system of claim 1, wherein the first observed variable is a genus that is significantly difference in abundance between the first intestinal flora data and the second intestinal flora data.

4. The system of claim 1, wherein the calculation unit uses a logistic regression model, when the second observed variables are binary categorical variables.

5. The system of claim 1, wherein the people with the one or more diseases include people who currently have the one or more diseases and/or people who have previously had the one or more diseases.

6. The system of claim 1, wherein the first creation unit further comprises a selection unit that selects the one or more genera using effect sizes as indexes.

7. The system of claim 1, wherein the second creation unit extracts a measurement equation model that shows influence of the latent variables on the first observed variable, from the association model, and
sets each parameter value of the measurement equation model to each parameter value of the score estimation model.

8. The system of claim 7, wherein the second creation unit further comprises a learning unit that learns each of the parameter values of the score estimation model using the first intestinal flora data and the second intestinal flora data.

9. The system of claim 1, wherein, when the one or more diseases are plural,
the first creation unit creates the association models for each of the diseases,
the second creation unit creates the score estimation models for each of the diseases, and
the calculation unit calculates the risks for each of the diseases or the risk for the plurality of diseases.

10. The system of claim 1, wherein, when the one or more diseases are plural, the second observed variables are plural,
the first creation unit adds another latent variable relating to the plurality of second observed variables to the association model,
the calculation unit to estimate score of the other latent variable from the estimated score, and to calculate the user's risk for the plurality of diseases using the parameters being from the other latent variable to the second observed variables and the score of the other latent variable.

11. A disease evaluation index calculation method, using a computer, the method comprising:
inputting one or more diseases that a user wants to evaluate whether it is at risk for into the computer;
extracting a first intestinal flora data of healthy people and a second intestinal flora data of people with the one or more diseases, using a first database that stores intestinal flora data being results of analyzing stool samples of a plurality of subjects, a second database that stores result data of a questionnaire on the one or more diseases for the plurality of subjects, and predetermined extraction conditions;
inputting the first intestinal flora data, the second intestinal flora data, and the result data of the questionnaire, and creating an association model including a first observed variable for one or more genera that express a difference in the first intestinal flora data and the second intestinal flora data, a second observed variable representing a morbidity status of the one or more diseases, one or more latent variables relating to the first observed variable and the second observed variable, and parameters being from the one or more latent variables to the first and the second observed variables and/or being between the latent variables;
creating a score estimation model including the first observed variable as an explanatory variable, and a score of the latent variable as an objective variable;
inputting the user's intestinal flora data to the explanatory variable of the score estimation model, and estimating the score of the latent variable; and
calculating the user's risk for the one or more diseases, using parameters being from the one or more latent variables to the second observed variables and/or being between the latent variables, and the estimated score.

12. A program for calculating a disease evaluation index, executed by a computer, the program comprising:
an input step of inputting one or more diseases that a user wants to evaluate whether it is at risk for into the computer;
an extraction step of extracting a first intestinal flora data of healthy people and a second intestinal flora data of people with the one or more diseases, using a first database that stores intestinal flora data being results of analyzing stool samples of a plurality of subjects, a second database that stores result data of a questionnaire on the one or more diseases for the plurality of subjects, and predetermined extraction conditions;
a first creation step of inputting the first intestinal flora data, the second intestinal flora data, and the result data of the questionnaire, and creating an association model including a first observed variable for one or more genera that express a difference in the first intestinal flora data and the second intestinal flora data, a second observed variable representing a morbidity status of the one or more diseases, one or more latent variables relating to the first observed variable and the second observed variable, and parameters being from the one or more latent variables to the first and the second observed variables and/or being between the latent variables;
a second creation step of creating a score estimation model including the first observed variable as an explanatory variable, and a score of the latent variable as an objective variable;
an estimation step of inputting the user's intestinal flora data to the explanatory variable of the score estimation model, and estimating the score of the latent variable; and
a calculation step of calculating the user's risk for the one or more diseases, using parameters being from the one or more latent variables to the second observed variables and/or being between the latent variables, and the estimated score.
